# EUROPEAN PATENT SPECIFICATION

(11) **EP 2 780 702 B1**
(45) Date of publication and mention of the grant of the patent: **14.02.2018**
(21) Application number: 12849801.1
(22) Date of filing: 19.11.2012
(51) Int. Cl.: G01N 33/483, B82Y 15/00, B82Y 40/00, G01N 27/327

(54) **MOLECULAR JUNCTION PLATFORM AND METHOD OF FABRICATING SUCH A PLATFORM**
MOLEKULARE VERBINDUNGSPLATTFORM UND VERFAHREN ZUR HERSTELLUNG EINER SOLCHEN PLATTFORM
PLATEFORME DE JONCTION MOLÉCULAIRE ET MÉTHODE DE FABRICATION D'UNE TELLE PLATEFORME

(30) Priority: 18.11.2011 SE 1151096
(43) Date of publication of application: 24.09.2014
(73) Proprietor: Leifer, Klaus, 752 36 Uppsala (SE)
(72) Inventor: Leifer, Klaus, 752 36 Uppsala (SE); Jafri, S. Hassan M., 753 12 Uppsala (SE)
(74) Representative: Bjerkéns Patentbyrå KB (Gävle)
(86) International application number: PCT/SE2012/051274
(87) International publication number: WO 2013/074037

(56) References cited:
- WO-A1-2006/088425
- WO-A1-2011/056501
- WO-A1-2011/056501
- JP-A- 2004 172 270
- JP-A- 2008 192 712
- S. HASSAN M. JAFRI ET AL: "Control of junction resistances in molecular electronic devices fabricated by FIB", MICROELECTRONIC ENGINEERING, vol. 88, no. 8, 5 December 2010 (2010-12-05), pages 2629-2631, XP055204694, ISSN: 0167-9317, DOI: 10.1016/j.mee.2010.11.040
- ANDREAS WALLNER ET AL: "Formation and NMR Spectroscopy of [omega]-Thiol Protected [alpha],[omega]-Alkanedithiol-Coated Gold Nanoparticles and Their Usage in Molecular Charge Transport Junctions", LANGMUIR, vol. 27, no. 14, 19 July 2011 (2011-07-19) , pages 9057-9067, XP055154973, ISSN: 0743-7463, DOI: 10.1021/la2019007
- Laetitia Bernard: "Expanding the horizon of molecular electronics via nanoparticle assemblies", , 1 January 2006 (2006-01-01), XP055205119, DOI: 10.5451/unibas-004166005 Retrieved from the Internet: URL:http://dx.doi.org/10.5451/unibas-00416 6005 [retrieved on 2015-07-27]
- ANDREAS WALLNER ET AL.: 'Formation and NMR Spectroscopy of ù-Thiol Protected a,u- Alkanedithiol-Coated Gold Nanoparticles and Their Usage in Molecular Charge Transport Junctions' LANGMUIR vol. 27, no. 14, 2011, pages 9057 - 9065, XP055154973

## Description

### FIELD OF THE INVENTION

The invention relates to a sensor for sensing gas, liquids and/or biomolecules comprising a molecular junction platform and a method of fabricating a molecular junction platform.

### BACKGROUND OF THE INVENTION

With the idea to create stable and reproducible metal-few-molecule junctions, we have previously created a platform where gold nanoparticles were dielectrophoretically trapped in between gold nanoelectrodes with the nanoelectrode surfaces functionalised by mono- and di-thiol terminated molecules (Jafri, S.H.M. et al., Nanotechnology 21, 435204 (2010)). However, these experiments showed a large variation of electrical properties between the different devices related to uncontrolled variations in the chemical binding between the molecules and the gold surfaces. This situation changed clearly, after functionalising the gold nanoparticles directly with alkanedithiol molecules (Wallner, A. et al., Langmuir 27, 9057-9067 (2011)). In this experiment, the dangling thiol group was protected using trityl protective groups, preventing so-called backbiting and oxidation. After trapping the functionalised nanoparticles into the nanoelectrode gap, the trityl group was removed using an acid treatment and thus the free dangling thiol could bind to the counter gold surface, i.e. a neighbouring nanoparticle. As a result, the junction in the nanoparticle bridge platform showed a high reproducibility and the junction characteristics corresponds to conduction through typically five to several tens of molecules. We could measure clear differences in conduction comparing i.e. electron transport through octane-dithiol and hexane-dithiol.

WO2006/088425 discloses a single electron transistor device for use in a sensor for detection of single molecules and a method of fabricating such a device.

Jafri, S.H.M. et al., Microelectronic Engineering 88, 2629-2631 (2011) disclose a molecular junction platform and a method for fabricating such a platform.

However, in order to obtain molecular junctions which could prove useful in for example sensing applications, it is desirable to develop a flexible and stable molecular junction platform using molecules with properties suitable for such applications.

### SUMMARY OF THE INVENTION

An object of the present invention is to provide an, in at least some aspect, improved gas sensor comprising a molecular junction platform and an improved method of fabricating a molecular junction platform and such a gas sensor. This object is achieved by a gas sensor according to claim 1 and a method of fabricating a molecular junction for a gas sensor according to claim 5. Preferred embodiments are disclosed in the dependent claims.

According to the invention, a molecular junction platform is provided which is stable in operation at ambient conditions, reproducible and versatile, and in which the electronic properties are dominated by the electronic properties of the molecules. The molecular junction platform has the advantages of being small and easy to upscale to a very large amount of nanoelectrodes. The molecules may according to the invention be exchanged and the platform therefore provides flexibility since the same platform may be adapted to be sensible to different kinds of gases, liquids, biomolecules, etc. The platform is therefore highly suitable for use in a sensor. Moreover, the platform can be easily incorporated in electronic circuits since the method of fabrication is compatible with standard fabrication techniques for electronic circuits.

According to one aspect of the invention, the invention relates to a gas sensor comprising a molecular junction platform according to the invention. Such a gas sensor is very sensitive and molecules in gaseous form can be sensed in very low concentrations in the environment surrounding the platform. The sensor is stable over time, and the sensitivity of the sensor can simply be increased by increasing the number of molecular junction platforms on a single chip. The small size of the sensor makes it very useful for detection of gases in limited and/or confined spaces down to a few hundred micrometers in size. Examples are small tubes where the sensor is so small that it does not hinder the flow, gas sensors in a lab-on-a-chip, in clothes, in safety and rescue. The electronic response of the sensor according to the invention is dominated by the response of electronic signature of individual sensing molecules, as opposed to a larger molecular sensor comprising thousands to billions of molecules, wherein the electronic response is due to the statistic average over the molecule population and is often dominated by statistical variations in the entire population of molecules.

### BRIEF DESCRIPTION OF THE DRAWINGS

The present invention will now be described in detail with reference to the attached drawings:
- Figure 1: is a schematic drawing of a molecular junction platform according to the invention,
- Figure 2: is a diagram showing results from electrical measurements on the molecular junction platform,
- Figure 3: is a diagram showing *I*-*V* characteristics for a molecular junction platform according to the invention,
- Figure 4: is a diagram showing the current through a gas sensor according to the invention, and
- Figure 5: is a diagram showing the current through a gas sensor according to the invention.

### DETAILED DESCRIPTION OF EMBODIMENTS OF THE INVENTION

The term "nanogap", as used herein, refers to a gap of less than 1 µm. The term "nanoelectrodes" refers to an electrode less than 1 µm wide. The term "nanoparticle" refers to a particle with a size of 1 nm to about 500 nm.

The term "biomolecules", as used herein, refers to molecules (e.g. proteins, amino acids, peptides, polynucleotides, nucleotides, carbohydrates, sugars, lipids, nucleoproteins, glycol-proteins, lipoproteins, steroids, etc.) whether naturally-occurring or artificially created (e.g., by synthetic or recombinant methods) that are commonly found in cells and tissues. Specific classes of biomolecules include, but are not limited to, enzymes, receptors, neurotransmitters, hormones, cytokines, cell response modifiers such as growth factors and chemotactic factors, antibodies, vaccines, haptens, toxins, interferons, ribozymes, anti-sense agents, plasmids, DNA, and RNA.

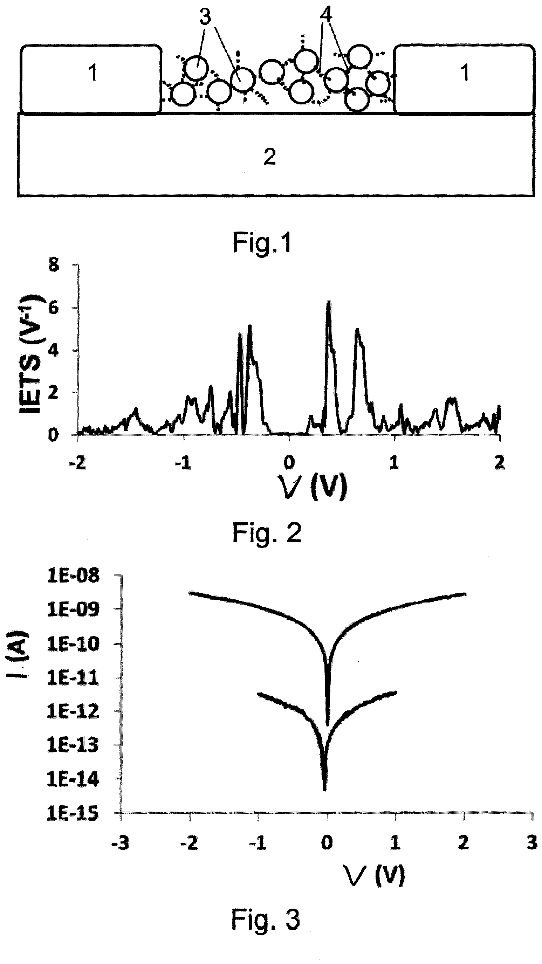

A molecular junction platform according to the invention is schematically drawn in Fig. 1. The platform comprises two nanoelectrodes 1 situated on a substrate 2, at least one nanoparticle 3, here a number of nanoparticles arranged in a three-dimensional network in between the nanoelectrodes, and molecules 4 connecting the nanoparticles 3 to the nanoelectrodes 1 and to each other. The nanoelectrodes 1 can be in the form of a thin film or in the form of a thin wire and can be made of gold or other conducting metals or oxides. The substrate 2 is preferably an oxidised silicon wafer, but also other substrates can be used, such as pores. The molecules 4 are preferably molecules suitable for sensing applications. The nanoelectrodes can be electrically connected to macroscopic devices using contact plates with a width of several tens of micrometers.

For fabrication of the molecular junction platform, one or more nanoelectrode pairs are prepared on a substrate, such as an oxidised silicon wafer, using established microfabrication techniques such as electron beam lithography, photolithography and/or focused ion beam milling. The nanoelectrodes of each nanoelectrode pair are separated by a nanogap which is less than 1 µm wide, preferably less than 100 nm wide, and even more preferably less than 50 nm wide. A typical nanogap spacing is between 1 nm and 10 nm. A large number of pairs of nanoelectrodes may be fabricated on the same substrate. Nanoparticles functionalised with a first set of molecules are then moved into the nanoelectrode nanogap using e.g. dielectrophoretic trapping techniques. Alternative techniques may be applied to move the nanoparticles into the nanoelectrode nanogap. The trapped nanoparticles arrange in a three-dimensional matrix in the nanogap separating the nanoelectrodes. In a next step, the first set of molecules are removed in a place exchange, and replaced by a second set of molecules, for example sensing molecules suitable for sensing applications. Sensing molecules can be defined as molecules that change their electronic structure due to the presence of gases in such a way that their electrical properties when connected to a bias voltage change. Place exchange of molecules is a decisive step since it allows the use of a wide variety of molecules in the nanoelectrode nanogap. A molecular junction is established since the sensing molecules connect at both ends to the nanoparticles and/or to the nanoelectrodes, creating a network connecting the two nanoelectrodes. The electronic response of the junction is dominated by the electronic properties of the sensing molecule.

The nanoparticles used may be for example gold, silver, platinum or semiconductor nanoparticles. The electronic properties of molecules are strongly affected by the nanoparticle material properties. In a preferred embodiment, gold nanoparticles are used. These can have a size from about 1 nm to about 500 nm, preferably less than 100 nm. Gold nanoparticles exhibit superior chemical stability and do not oxidise at room temperature, although gold atoms can diffuse at room temperature. Thiol (-SH), amino (-NH₂) and selenol (-SeH) terminated molecules can be chemisorbed on gold surfaces.

Also silver nanoparticles of sizes from 1 nm to about 100 nm may be used. Silver nanoparticles tend to oxidise at room temperature and they are therefore more difficult to use in electronics or sensors. Thiol and amino terminated molecules can be chemisorbed on silver nanoparticles

Platinum nanoparticles in a size range of 2-10 nm may also be used. The chemical bonding between thiol and amino groups and platinum surfaces is very good. Platinum is also very stable at room temperature.

Also other metallic and semiconductor nanoparticle materials may be considered, for example aluminium, titanium, II/VI or III/V semiconductors depending on the application of the molecular junction platform.

The nanoparticles are functionalised using a molecule which at least on one end comprises a group which binds to the nanoparticles, such as a thiol or an amino group. The functionalised nanoparticles are trapped in the nanogap between the nanoelectrodes by using e.g. dielectrophoresis. The functionalising molecules act as spacers between the nanoparticles, preventing them from aggregating and fusing. The functionalising molecules should function at least as spacers and therefore the following criteria should be fulfilled: 1) the molecules must be rigid i.e. stick out by some distance; and 2) they must cover most of the nanoparticle surface.

Examples of sensing molecules suitable for use in a gas sensor according to the invention are molecules comprising phenyl groups and end groups, such as thiol (-SH), amino (-NH₂), carboxyl (-COOH) or selenol (-SeH) groups, which end groups can bind to the nanoparticles and/or nanoelectrodes. Examples of such molecules are biphenyldithiol (BPDT) and Tour's compound. Other interesting molecules are organic molecules that contain either alkane chains or cycloalkane chains or both in addition to the above-mentioned end groups. An example is octanedithiol. The organic molecules may contain other atoms or ions such as iron, oxygen, chlorine, phosphorous, etc. The additional atoms or ions increase the gas sensitivity and/or selectivity of the sensor when the molecules in the molecular junction platform are exposed to one or a mixture of gases. Examples of interesting molecules are ferrocenedithiol and other metal-containing aromatic molecules which can change their electronic properties in presence of gases or liquids.

In one embodiment, the nanoparticles are functionalised using a molecule which comprises two groups that can bind to the nanoparticles. The functionalised nanoparticles are trapped in the nanogap between the nanoelectrodes. The functionalising molecules act as spacers between the nanoparticles, preventing them from aggregating, but on the same time also establishes a network connecting the nanoelectrodes. In a next step, the molecules are removed in a place exchange, and replaced by molecules suitable for sensing applications, i.e. molecules that react sensitively to electron donating or electron accepting gases.

In one embodiment, the nanoparticles are functionalised using a molecule which on one end comprises a protective end group, preventing this end from binding to the nanoparticles. An example of such a molecule is an alkane-dithiol, which on one end is protected by a trityl group. The thus functionalised and protected nanoparticles are trapped in the nanogap between the nanoelectrodes with the alkane-dithiols linked only on one side to the nanoparticles. The alkane-dithiols here act as spacers between the nanoparticles, preventing them from aggregating. In a next step, the alkane-dithiols containing protective trityl groups may be removed in a place exchange, and replaced by molecules suitable for sensing applications, i.e. molecules that react sensitively to electron donating or electron accepting gases. Once the molecules comprising a protective end group are replaced by sensing molecules, a molecular junction is established since the sensing molecules connect at both ends to the nanoparticles and/or to the nanoelectrodes, creating a network connecting the two nanoelectrodes.

The use of a molecule comprising a protective end group preventing one end of the functionalising molecule to connect to the nanoparticles/nanoelectrodes, has the advantage of being more easily replaced in a place exchange than molecules connected at both ends to a nanoparticle. Other examples of protective end groups are cyanoethyl and trimethylsilylethyl. However, any atom or chain of atoms that can be attached to the one thiolated end of molecules to protect them from forming a chemisorbed junction at the both end of molecules through backbiting or disulfide bonds may be used as a protective end group. After trapping, these additional atoms are removed to form chemisorbed metal-molecule junctions at both ends of the molecules with adjacent nanoelectrode or nanoparticle surfaces. The use of "few" molecules makes it possible to use this platform also for thermoelectrical energy generation using the Seebeck effect. The Seebeck effect should be highly efficient when molecules are used in a junction. A device for thermoelectrical energy generation could include at least one molecular junction platform according to the invention, preferably a large number of such platforms.

The molecular junction platform according to the invention is used as a sensor for sensing gases. In particular, the molecular junction platform is useful in a gas sensor. The sensor can comprise one or more molecular junction platforms. The sensing molecular junction can be as small as about 1 nm. The nanoelectrodes and the nanogap between them have a size range between some nanometers and the 100 nm range. Thus, this sensor can be used to measure gases in very small units. An alternative is to integrate the sensor into other microcircuits, i.e. to integrate it directly on a chip. Another variant consists in producing large amounts of sensors on one single wafer. This can either reduce production cost or it can be used to fabricate extremely sensitive sensors. Using modern lithography techniques, in principle, the same integration densities of these molecular junction platforms can be achieved as for modern microprocessors, i.e. millions to billions of devices on a chip can be reached.

The gas sensor according to the invention has experimentally been found to be useful for sensing nitrogen dioxide, carbon monoxide, benzene, ammonia, and alcohol such as ethanol in a sensitivity range down to 100 ppm. Potentially the gas sensor can be used to sense inorganic molecules in gaseous state such as ammonia, arsenic pentafluoride, arsine, boron trichloride, boron trifluoride, bromine, carbon disulfide, carbon monoxide, carbon tetrachloride, chlorine, chlorine dioxide, cyanogen chloride, diborane, dichlorosilane, fluorine, germane, hydrazine, hydrogen, hydrogen bromide, hydrogen chloride, hydrogen sulphide, and nitrogen dioxide. The gas sensor could also be useful for sensing organic molecules in gaseous state, such acetic acid, acetone, acetonitrile, acetylene, acrolein (acryl-aldehyde), acrylic acid, acrylonitrile, allyl alcohol, allyl chloride, anisole, biphenyl, butadiene, butane, butanol, butene, butyl acetate, cellosolve acetate, chlorobutadiene, chloroethanol, chloroform, chlorotrifluoroethylene, cumene, cyclohexane, cyclopentane, dibromoethane, dibutylamine, dichlorobutene, dichloroethane, dichlorofluoroethane, dichloropentadiene, diethyl benzene, diethyl sulphide, difluorochloroethane, difluoroethane, dimethyl ether, dimethylamine, epichlorohydrin, ethane, ethyl acetate, ethyl benzene, ethyl chloride, ethyl chlorocarbonate, ethyl ether, ethylene, ethylene oxide, formaldehyde, trichlorofluoromethane, dichlorodifluoromethane, chlorodifluoromethane, trichlorotrifluoroethane, dichlorotetrafluoroethane, dichlorotrifluoromethylmethane, heptane, hexane, and hexene.

With the gas sensor according to the invention, the presence of gases can be monitored or detected via a change in the noise level, i.e. the fluctuation amplitude, in the signal, or via a change in the electrical impedance of the sensor. The changes can be displayed in terms of a digital or analogue output signal using electronic circuitry connected to the sensor. The adsorption or desorption of gas molecules on the sensing molecules of the sensor can be seen either in current response at an applied voltage or in voltage response at an applied current.

Storage recipes are crucial for using molecular electronic devices in applications. Storing tests where molecular junction devices according to the invention as well as pure nanoelectrodes were stored a) under ambient conditions (air), b) in water, c) in tetramethylethylenediamine (TMEDA), d) in toluene, and e) in tetrahydrofuran (THF) for four weeks, with evaluations of electrical properties (resistance) and structure (scanning electron microscopy, SEM) every week, have revealed that the pure nanoelectrode structure is most stable in pure water storage as well as in toluene. The molecular junction devices, according to the tests, have a stable electrical performance when storing in TMEDA.

### Electrical measurements

Electrical contacts can be connected to the nanoelectrodes to measure the electrical properties of the molecular junction. The junction is designed such that the electrical properties of the molecules dominate the electrical properties of the junctions. The use of sensing molecules that change their electronic properties in the presence of gases, liquids and biomolecules enables the sensing action. When a current flows across the junction, the current will change when the molecule is exposed to different environments. The use of different sensing molecules that show different electrical signatures in the presence of different gases, liquids and biomolecules makes it possible to target different molecular species when using different sensing molecules in different devices. Figure 2-3 show results from electrical measurements. Figure 3 shows *I*-*V* curves (current *I* plotted as a function of voltage V) after placement of molecules in a nanoparticle-nanoelectrode platform (lower curve) and after formation of a stable metal-molecule junction, i.e. a molecular junction platform (upper curve). An alternative to *I*-*V* curves is the measurement of inelastic tunnelling spectroscopy (IETS) that gives signatures of the vibrational states of the sensing molecules.

The IETS measurements involve:
to contact the molecular junction platform according to the invention at liquid helium temperatures;
to have built a stable molecular junction platform, where the junctions are well formed for longer periods and for different temperatures;
to have well formed reproducible junctions in the molecular junction platform; and
to have sensitive electronic measurement equipment capable of working at very low currents and low signal to noise ratios.

Results from IETS measurements on the molecular junction platform using octanedithiol (ODT) molecules are shown in figure 2. The peaks that can be attributed to vibrational levels of the ODT molecule show the signature of ODT.

Measurements of vibrations of a few (some ten) molecules can be equally used as a sensor principle since the vibrations should change in a well defined way, when e.g. gas molecules are attached to the molecule in the molecular junction platform.

### Example 1

Place exchange was applied in a molecular junction platform according to the invention. The platform in this example comprised gold nanoelectrodes separated by a nanogap of less than 50 nm and gold nanoparticles functionalised with a first set of functionalising molecules being trityl-protected octanethiol molecules. A substrate comprising several of said platforms was immersed in a solution of 1 mM of BPDT in toluene for 48 hours. Place exchange was evidenced by electrical measurements and Raman spectroscopy. By alternately immersing the substrate in a solution containing BPDT and a solution containing octanethiol, it was found that it is possible to repeat the place exchange step several times with good results. Potentially, different sensing molecules could be used and the platform is therefore very versatile.

### Example 2

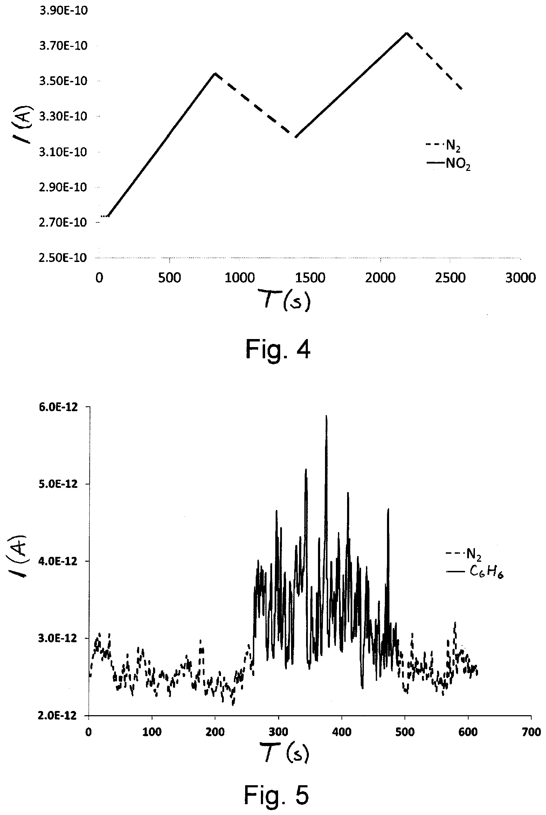

A gas sensor according to an embodiment of the invention was fabricated using the described molecular junction platform. In this example, a molecular junction platform with gold nanoelectrodes separated by a nanogap of approximately 19 nm and gold nanoparticles was used. The sensing molecules were 4,4'-biphenyldithiol molecules. The current *I* flowing across the platform was measured as a function of time T, as shown in Fig. 4. The solid line (linear regression fit) represents change in measured current at a constant applied voltage bias when the sensing molecules in the molecular junction platform were exposed to a continuous flow of a gas containing 100 ppm NO₂ in N₂ and the dotted line (linear regression fit) represents change in measured current at a constant applied bias when pure N₂ was flushed to remove NO₂ in the surroundings of the sensor. The current can be seen to increase when gas molecules are adsorbed on the sensing molecules of the sensor, and to decrease as the gas molecules are desorbed.

### Example 3

A gas sensor according to another embodiment of the invention was fabricated using the described molecular junction platform. In this example, a molecular junction platform with gold nanoelectrodes separated by a nanogap of approximately 19 nm and gold nanoparticles was used. The sensing molecules were 1,8-octanedithiol molecules. Fig. 5 shows the current *I* across the sensor as a function of time *T*. The solid line represents fluctuations in measured current at a constant applied bias when the sensing molecules in the molecular junction platform were exposed to benzene (C₆H₆) molecules and the dotted line represents fluctuation in measured current at a constant applied bias when N₂ was flushed to remove benzene in the surroundings of the gas sensor. As can be seen from the diagram, the fluctuation amplitude increases as benzene molecules are adsorbed on the sensing molecules of the sensor and decreases as the benzene molecules desorb.

## Claims

1. A gas sensor comprising at least one platform comprising two nanoelectrodes (1) with a width of less than 1 µm separated by a nanogap of less than 1 µm, and at least one nanoparticle (3) with a size of 1-500 nm situated between the nanoelectrodes (1),
***characterised in***
**that** the platform further comprises at least two molecules (4) connecting the at least one nanoparticle (3) to the nanoelectrodes (1), creating a network connecting the two nanoelectrodes (1) such that a molecular junction is established, wherein the at least two molecules (4) are sensing molecules suitable for sensing gas.

2. A gas sensor according to claim 1, wherein the platform comprises several nanoparticles (3) arranged in a three-dimensional network connected by said molecules (4).

3. A gas sensor according to claim 1 or 2, wherein the sensing molecules each include at least one phenyl group or one alkane group.

4. A gas sensor according to claim 2 or 3, wherein the sensing molecules each include at least one thiol group, one amino group, one carboxyl group or one selenol group.

5. A method of fabricating a molecular junction platform for a gas sensor comprising two nanoelectrodes (1) with a width of less than 1 µm separated by a nanogap of less than 1 µm, at least one nanoparticle (3) with a size of 1-500 nm situated between the nanoelectrodes (1) and at least two molecules (4) connecting the at least one nanoparticle (3) to the nanoelectrodes (1), the method comprising the steps of:
- preparation of at least one nanoparticle (3) functionalised with a first set of molecules;
- trapping of the at least one functionalised nanoparticle (3) in a nanogap between two nanoelectrodes (1) on a substrate (2);
***characterised in*** the step of:
- subsequently to trapping of the at least one functionalised nanoparticle (3), carrying out at least one place exchange reaction replacing the first set of molecules by a second set of molecules (4), so that the second set of molecules (4) creates a network connecting the nanoelectrodes (1) and the at least one nanoparticle (3) such that a molecular junction is established, wherein the second set of molecules (4) are sensing molecules suitable for sensing gas.

6. A method of fabricating a molecular junction platform according to claim 5, the method comprising fabricating a molecular junction platform comprising several nanoparticles (3) arranged in a three-dimensional network connected by said molecules (4).

7. A method of fabricating a molecular junction platform according to claim 5 or 6, wherein the first set of molecules comprises molecules including on one end a protective end group preventing the protected end from binding to a surface.

8. A method of fabricating a molecular junction platform according to claim 7, wherein the protective end group preferably is a trityl group, a cyanoethyl group or a trimethylsilylethyl group.

9. A method of fabricating a molecular junction platform according to any one of claims 5-8, wherein the sensing molecules each include at least one phenyl group.

10. A method of fabricating a molecular junction platform according to any of claims 5-9, wherein the sensing molecules each include at least one thiol group, one amino group or one selenol group.

11. A method of fabricating a molecular junction platform according to any of claims 5-10, wherein the trapping of the functionalised nanoparticles (3) is done using dielectrophoresis.

## Patentansprüche

1. Gassensor, der mindestens eine Plattform beinhaltet bestehend aus zwei Nanoelektroden (1) mit einer Breite von weniger als 1 µm, die durch einen Nanospalt von weniger als 1 µm getrennt sind, und wenigstens einem Nanoteilchen (3) mit einer Grösse von 1-500 nm, das zwischen den Nanoelektroden positioniert ist (1),
**dadurch gekennzeichnet,**
**dass** die Plattform wenigstens zwei Moleküle beinhaltet, die wenigstens ein Nanoteilchen (3) mit den zwei Elektroden verbinden, so dass ein molekularer Übergang geschaffen wird, wobei wenigstens zwei Moleküle (4) Sensormoleküle sind, die zum Abfühlen von Gasen geeignet sind.

2. Gassensor nach Anspruch 1, wobei die Plattform mehrere Nanoteilchen enthält (3), die dreidimensional angeordnet sind, verbunden durch die genannten Moleküle (4).

3. Gassensor nach Anspruch 1 oder 2, worin jedes der Sensormoleküle wenigstens eine Phenylgruppe oder eine Alkangruppe beinhalten.

4. Gassensor nach Anspruch 2 oder 3, worin jedes der Sensormoleküle wenigstens eine Thiolgruppe, eine Aminogruppe, eine Carboxylgruppe oder eine Selenolgruppe beinhalten.

5. Verfahren zur Fabrikation einer molekularen Übergangsplatform für einen Gassensor, die aus zwei Nanoelektroden (1) mit einer Breite von weniger als 1 µm besteht, getrennt durch eine Nanospalt von weniger als 1µm, wenigstens einem Nanoteilchen (3) mit einer Größe von 1-500 nm, das zwischen den Nanoelektroden (1) positioniert ist und wenigstens zwei Molekülen (4), die das Nanoteilchen (3) mit den Nanoelektroden (1) verbinden, wobei das Verfahren aus den Schritten besteht:
- Präparation von wenigstens einem Nanoteilchen (3), das mit einem Satz von Molekülen funktionalisiert ist;
- Trapping von wenigstens einem funktionalisierten Nanoteilchen (3) in eine Nanolücke zwischen den zwei Nanoelektroden (1) auf einem Substrat (2);
**gekennzeichnet durch** den Schritt von:
- aufeinanderfolgendes Trapping von wenigstens einem funktionalisierten Nanoteilchen (3), das wenigstens eine Ligandenaustauschreaktion ausführt und dabei den ersten Satz von Molekülen durch einen zweiten Satz von Molekülen (4) ersetzt, so dass der zweite Satz von Molekülen (4) ein Netzwerk bildet, das die Nanoelektroden (1) und wenigstens ein Nanoteilchen (3) verbindet, so dass ein molekularer Übergang gebildet wird, worin der zweite Satz von Molekülen (4) Sensormoleküle sind, die geeignet sind zum Abfühlen eines Gases.

6. Verfahren zur Fabrikation einer molekularen Übergangsplatform nach Anspruch 5, das Verfahren bestehend aus der Fabrikation einer molekularen Übergangsplatform bestehend aus mehreren Nanoteilchen (3), die in einem 3-dimensionalen Netzwerk geordnet sind verbunden durch die genannten Moleküle (4).

7. Verfahren zur Fabrikation einer molekularen Übergangsplatform nach Anspruch 5 oder 6, wobei der erste Satz von Molekülen aus Molekülen besteht, einschließlich an einem ihrer Enden eine schützende Endgruppe, die verhindert, dass sich die geschützten Enden an eine Oberfläche binden.

8. Verfahren zur Fabrikation einer molekularen Übergangsplatform nach Anspruch 7, wobei die protektive Endgruppe vorzugsweise eine Tritylgruppe, eine Cyanoethylgruppe oder eine Trimethylsilylethylgruppe ist.

9. Verfahren zur Fabrikation einer molekularen Übergangsplatform nach einem der Ansprüche 5-9, wobei jedes der Sensormoleküle wenigstens eine Phenylgruppe beinhalten.

10. Verfahren zur Fabrikation einer molekularen Übergangsplatform nach Anspruch 9, wobei die Sensormoleküle jedes wenigstens eine Thiolgruppe, eine Aminogruppe oder eine Selenolgruppe einschliessen.

11. Verfahren zur Fabrikation einer molekularen Übergangsplatform nach einem der Ansprüche 5-10, wobei das Trapping der funktionalisierten Nanoteilchen (3) durch Elektrophoresis durchgeführt wird.

## Revendications

1. Capteur de gaz comprenant au moins une plateforme comprenant deux nano-électrodes (1) avec une largeur de moins de 1 µm separées par un nano-espacement de moins de 1 µm, et au moins une nanoparticule (3) d'une taille de 1-500 nm positionnée entre les nano-électrodes (1),
**caractérisé en**
**ce que** la plateforme en plus comprends au moins deux molécules (4) connectant la au moins une nanoparticule (3) aux nano-électrodes (1) afin de créer un réseau connectant les deux nano-électrodes (1) de façon qu'une jonction moléculaire est établie, dans laquelle les au moins deux molécules (4) sont des molécules senseurs capables de detecter du gaz.

2. Capteur de gaz suivant la revendication 1, dans lequel la plateforme comprend plusieurs nanoparticules (3) arrangées dans un réseau tridimensionnel connectées par les dites molécules (4).

3. Capteur de gaz suivant la revendication 1 ou 2, dans lequel les molécules senseur chacune inclut au moins un groupe de phenyl ou un groupe d'alcane.

4. Capteur de gaz suivant la revendication 2 ou 3, dans lequel chaque molécule senseur inclut au moins un groupe de thiol, un groupe d'amino, un groupe de carboxyle ou un groupe de selenole.

5. Méthode de fabrication d'une plateforme à jonction moléculaire pour un capteur de gaz comprenant deux nano-électrodes (1) avec une largeur de moins de 1 µm separées par un nano-espacement de moins de 1 µm, au moins une nanoparticule (3) avec une taille de 1-500 nm positionnée entre les nano-électrodes (1) et au moins deux molécules (4) connectant la au moins une nanoparticule (3) aux nano-électrodes, la méthode comprenant les pas suivants :
- préparation d'au moins une nanoparticule (3) fonctionnalisée avec un premier ensemble de molécules;
- piégeage de la au moins une nanoparticule (3) dans un nano-espacement entre deux nano-électrodes (1) sur un substrat (2) ;
**caractérisée par** le pas suivant :
- après le piégeage d'au moins une nanoparticule (3) fonctionnalisée, exécutant au moins une réaction d'échange de ligands remplaçant le premier ensemble de molécules par un deuxième ensemble de molécules (4), afin que le deuxième ensemble de molécules (4) crée un réseau connectant les nano-électrodes (1) et la au moins une nanoparticule (3) de façon qu'une jonction moléculaire est établie, dans laquelle le deuxième ensemble de molécules (4) sont des molécules senseurs capables de detecter du gaz.

6. Méthode de fabrication d'une plateforme à jonction moléculaire suivant la revendication 5, la méthode comprenant la fabrication de plateforme à jonction moléculaire comprenant plusieurs nanoparticules (3) arrangées dans une réseau trois dimensionel connecté par les dites molécules (4).

7. Méthode de fabrication d'une plateforme à jonction moléculaire suivant la revendication 5 ou 6, dans laquelle le premier ensemble de molécule inclut des molécules qui, à un des deux points terminaux, comprennent un groupe protecteur empêchant que le point terminal protégé crée une liaison à une surface.

8. Méthode de fabrication d'une plateforme à jonction moléculaire suivant la revendication 7, dans laquelle le groupe protecteur est préférablement un groupe de trityle, de cyanoethyle ou de trimethyle-silyle-ethyle.

9. Méthode de fabrication d'une plateforme à jonction moléculaire suivant l'une quelconque des revendications 5-8, dans laquelle les molécules senseurs chacune inclut au moins un groupe de phenyle.

10. Méthode de fabrication d'une plateforme à jonction moléculaire suivant l'une quelconque des revendications 5-9, dans laquelle les molécules senseurs chacune inclut au moins un groupe de thiole, un groupe d'amine ou un groupe de selenole.

11. Méthode de fabrication d'une plateforme à jonction moléculaire suivant l'une quelconque des revendications 5-10, dans laquelle le piégeage de nanoparticules fonctionalisées (3) est faite par di-électrophorèse.
